# EUROPEAN PATENT APPLICATION

(11) **EP 4 520 747 A1**
(43) Date of publication of application: **12.03.2025**
(21) Application number: 23823990.9
(22) Date of filing: 16.06.2023
(51) Int. Cl.: C07C 69/65, C07D 303/40, C07D 305/12, C07D 301/14, C07D 307/33

(54) **DIFLUOROALDEHYDE, EPOXY, AND DERIVATIVE THEREOF**

(30) Priority: 17.06.2022 JP 2022097789
(71) Applicant: SynCrest Inc., Fujisawa-shi, Kanagawa 251-8555 (JP); University of The Ryukyus, Nakagami-gun, Okinawa 903-0213 (JP)
(72) Inventor: XU, Pengyu, Fujisawa-shi, Kanagawa 251-8555 (JP); YONEYAMA, Shin, Fujisawa-shi, Kanagawa 251-8555 (JP); ARIMITSU, Satoru, Nakagami-gun, Okinawa 903-0213 (JP)
(74) Representative: Meissner Bolte Partnerschaft mbB
(86) International application number: PCT/JP2023/022347
(87) International publication number: WO 2023/243700

(57) **Abstract**

An object of the present invention is to provide an aldehyde compound and an ester compound that both have difluoro at the α-position, a double bond or epoxy at the β-position, and a disubstituted alkene at the γ-position. Provided are difluoroaldehyde, epoxy, and derivatives thereof.

## Description

### Technical Field

The present invention relates to difluoroaldehyde, epoxy, and derivatives thereof.

### Background Art

NPL 1 discloses the synthesis of esters having difluoro at the α-position. This synthesis example has low yields.

NPL 2 and 3 disclose the synthesis of gemcitabine. In these synthesis examples, a disubstituted fluorinated nucleoside is used as the starting material; their yield and selectivity are low, and the reaction time is long. In these synthesis examples, a four-membered ring compound cannot be produced.

### Citation List

### Non-patent Literature

NPL 1: ACS Sustainable Chemistry & Engineering, 2020, 8, 6533-6542
NPL 2: Tetrahedron, 2019, 75, 1203-1213
NPL 3: Carbohydrate Research, 2015, 406, 71-75

### Summary of Invention

### Technical Problem

An object of the present invention is to provide an aldehyde compound and an ester compound that both have difluoro at the α-position, a double bond or epoxy at the β-position, and a disubstituted alkene at the γ-position.

### Solution to Problem

The present inventors conducted extensive research to achieve the object above and succeeded in synthesizing an aldehyde compound and an ester compound having a difluoro at the α-position, a double bond or epoxy at the β-position, and a disubstituted alkene at the γ-position.

The present inventors conducted further research based on the finding and completed the present invention.

Specifically, the present invention encompasses the following difluoro compounds.

### Item 1.

A compound represented by the following formula (1): 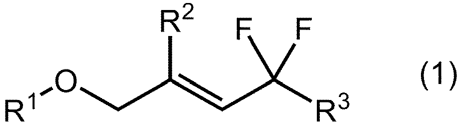 wherein
R¹ and R² may be the same or different, and each represents an alkyl group, an aryl group, a group having an ether bond, an ester group, an acetyl group, a benzoyl group, an alkyl alcohol group, an amide group, or a nitrile group, and
R³ represents an aldehyde group or an ester group.

### Item 2.

A compound represented by the following formula (2): 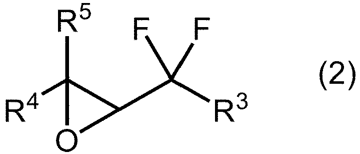 wherein
R⁴ and R⁵ may be the same or different, and each represents a hydrogen atom, an alkyl group, an aryl group, a group having an ether bond, an ester group, an acetyl group, a benzoyl group, an alkyl alcohol group, an amide group, or a nitrile group, and
R³ represents an aldehyde group or an ester group.

### Item 3.

A compound represented by the following formula (3): 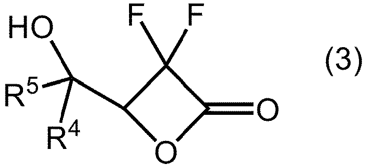 wherein
R⁴ and R⁵ may be the same or different, and each represents a hydrogen atom, an alkyl group, an aryl group, a group having an ether bond, an ester group, an acetyl group, a benzoyl group, an alkyl alcohol group, an amide group, or a nitrile group.

The aldehyde compound and ester compound of the present invention, which have difluoro at the α-position, a double bond or epoxy at the β-position, and a disubstituted alkene at the γ-position, can be easily derived into five-membered or four-membered ring compounds etc., and can expand the diversity of medium molecule precursors.

### Advantageous Effects of Invention

The present invention provides an aldehyde compound and an ester compound that both have difluoro at the α-position, a double bond or epoxy at the β-position, and a disubstituted alkene at the γ-position.

### Description of Embodiments

The present invention is described in detail below.

In the present specification, the terms "comprise" and "contain" include the concepts of comprising, consisting essentially of, and consisting of.

In the present specification, a numerical range indicated by "A to B" means "A or more and B or less."

### [1] Difluoro Compound

The difluoro compound of the present invention includes a compound represented by the following formula (1).

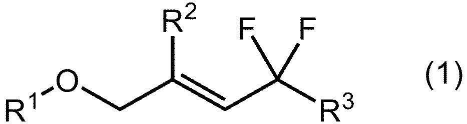

In formula (1), R¹ and R² may be the same or different, and each represents an alkyl group, an aryl group, a group having an ether bond, an ester group, an acetyl group (CH₃CO-), a benzoyl group (C₆H₅-C(=O)-), an alkyl alcohol group, an amide group, or a nitrile group (-C≡N).

In formula (1), R³ represents an aldehyde group (-CHO) or an ester group.

The difluoro compound of the present invention includes a compound represented by the following formula (2):

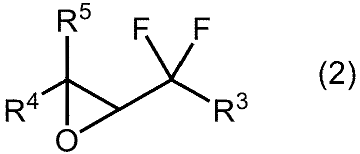

In formula (2), R⁴ and R⁵ may be the same or different, and each represents a hydrogen atom, an alkyl group, an aryl group, a group having an ether bond, an ester group, an acetyl group, a benzoyl group, an alkyl alcohol group, an amide group, or a nitrile group. R⁴ and R⁵ are preferably the same or different, and each represents an alkyl group, an aryl group, a group having an ether bond, an ester group, an acetyl group, a benzoyl group, an alkyl alcohol group, an amide group, or a nitrile group.

In formula (2), R³ represents an aldehyde group (-CHO) or an ester group.

The difluoro compound of the present invention includes a compound represented by the following formula (3).

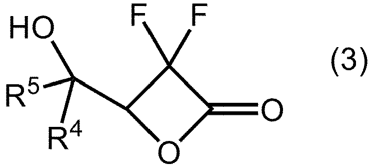

In formula (3), R⁴ and R⁵ may be the same or different, and each represents a hydrogen atom, an alkyl group, an aryl group, a group having an ether bond, an ester group, an acetyl group, a benzoyl group, an alkyl alcohol group, an amide group, or a nitrile group. R⁴ and R⁵ are preferably the same or different, and each represents an alkyl group, an aryl group, a group having an ether bond, an ester group, an acetyl group, a benzoyl group, an alkyl alcohol group, an amide group, or a nitrile group.

The aldehyde compound and ester compound of the present invention, which have difluoro at the α-position, a double bond or epoxy at the β-position, and a disubstituted alkene at the γ-position, can be easily derived into five-membered or four-membered ring compounds etc., and can expand the diversity of medium molecule precursors.

The alkyl group is preferably a linear, branched, or cyclic alkyl group. Specific examples include C₁₋₄ linear or branched alkyl groups, such as methyl, ethyl, n-propyl, isopropyl, n-butyl, isobutyl, sec-butyl, tert-butyl, and 1-ethylpropyl; C₁₋₁₈ linear or branched alkyl groups, such as n-pentyl, isopentyl, neopentyl, n-hexyl, isohexyl, 3-methylpentyl, n-heptyl, n-octyl, n-nonyl, n-decyl, n-undecyl, n-dodecyl, 5-propylnonyl, n-tridecyl, n-tetradecyl, n-pentadecyl, hexadecyl, heptadecyl, and octadecyl; and C₃₋₈ cyclic alkyl groups, such as cyclopropyl, cyclobutyl, cyclopentyl, cyclohexyl, cycloheptyl, and cyclooctyl.

The aryl group is preferably phenyl, biphenyl, naphthyl, dihydroindenyl, a 9H-fluorenyl group, or the like.

The group having an ether bond is not particularly limited.

The ester group is preferably a group with which an alkyl group as described above is bonded via an ester bond. The ester group is also represented by an alkoxycarbonyl group, an alkoxycarbonylalkyl group, or a carboxyalkyl group. The ester group is preferably a methyl ester group (COOMe), an ethyl ester group (COOEt), or the like. The ester group is preferably a 3-carboxypropyl group ((CH₂)₃COOH), a 3-methyloxycarbonylpropyl group ((CH₂)₃COOMe), or the like.

The alkyl alcohol group is not particularly limited.

The amide group is a primary amide (-NH₂), a secondary amide (-NHR¹), or a tertiary amide (-NR¹R²).

### [2] Method for Producing Difluoro Compound

### [2-1] Method for Producing Difluoro Compound Represented by Formula (1)

According to the following production method, a difluoro compound having the structure represented by formula (1) can be obtained from a β-unsaturated aldehyde or a β-unsaturated ester compound.

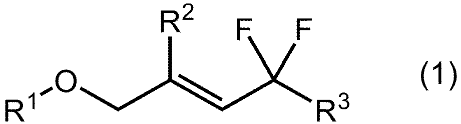

In formula (1), R¹ and R² may be the same or different, and each represents an alkyl group, an aryl group, a group having an ether bond, an ester group, an acetyl group (CH₃CO-), a benzoyl group (C₆H₅-C(=O)-), an alkyl alcohol group, an amide group, or a nitrile group (-C≡N).

In formula (1), R³ represents an aldehyde group or an ester group.

### β-unsaturated Aldehyde Compound and β-unsaturated Ester Compound (Substrate, Raw Material Compound)

A difluoro compound having the structure represented by formula (1) can be obtained from a β-unsaturated aldehyde compound or a β-unsaturated ester compound as a substrate by combining the known Parikh-Doering oxidation and Pinnick oxidation.

### Organic Solvent

The organic solvent is preferably at least one organic solvent selected from the group consisting of aprotic polar solvents, halogen solvents, ether solvents, ester solvents, hydrocarbon solvents, and aromatic solvents.

The aprotic polar solvents are preferably acetone, acetonitrile, N,N-dimethylformamide (DMF), dimethylsulfoxide (DMSO), and the like.

The halogen solvents are preferably dichloromethane (CH₂Cl₂), trichloromethane (CHCl₃), and the like.

The ether solvents are preferably tetrahydrofuran (THF), dioxane, diethyl ether (Et₂O), isopropyl ether (IPE), and the like.

The ester solvents are preferably ethyl acetate (AcOEt) and the like.

The hydrocarbon solvents are preferably n-hexane and the like.

The aromatic solvents are preferably toluene and the like.

The organic solvents are more preferably aprotic polar solvents, such as acetone, acetonitrile, DMF, or DMSO; ether solvents (polar solvents), such as THF, dioxane, or diethyl ether; or halogen solvents, such as dichloromethane or trichloromethane.

The amount of the organic solvent used is adjusted so that the substrate concentration (M (mol/L)) is preferably 0.01M to 5M, and more preferably 0.1M to 2M.

### Catalyst

The catalyst for use is preferably a primary amine organic catalyst, a secondary amine organic catalyst, or the like.

The amount of the catalyst for use as a concentration is preferably 5 to 40 mol%, and more preferably 10 to 30 mol%, relative to the substrate in molar ratio (when the substrate is taken as 100 mol%).

### Fluorinating Agent

The fluorinating agent for use is preferably at least one member selected from the group consisting of N-fluorobenzenesulfonimide (NFSI), 1-chloromethyl-4-fluoro-1,4-diazoniabicyclo[2.2.2]octane bis(tetrafluoroborate) (Selectfluor(R)), 1-fluoro-2,4,6-trimethylpyridinium tetrafluoroborate, 2,6-dichloro-1-fluoropyridinium tetrafluoroborate, and 2,6-dichloro-1-fluoropyridinium triflate; and more preferably N-fluorobenzenesulfonimide (NFSI).

The amount of the fluorinating agent for use is preferably 0.1 to 20 equivalents, and more preferably 0.9 to 5 equivalents, in molar ratio relative to the substrate (when the substrate is taken as 1 equivalent).

### Step of Producing Difluoro Compound Represented by Formula (1)

A difluoro compound represented by formula (1) can be produced from a β-unsaturated aldehyde compound or a β-unsaturated ester compound as a substrate (raw material compound) by combining the known Parikh-Doering oxidation and Pinnick oxidation.

The reaction temperature is preferably 0°C to 90°C, and more preferably 0°C to 60°C.

The reaction time is preferably 0.5 hours to 70 hours, and more preferably 10 hours to 60 hours.

### [2-2] Method for Producing Difluoro Compound Represented by Formula (2)

According to the following production method, a difluoro compound having the structure represented by formula (2) can be obtained from a difluoro compound represented by formula (1). In this production method, the combination of bases and the reaction temperature are adjusted to stabilize epoxy, thereby satisfactorily producing a difluoro compound having the structure represented by formula (2).

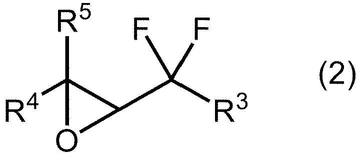

In formula (2), R⁴ and R⁵ may be the same or different, and each represents a hydrogen atom, an alkyl group, an aryl group, a group having an ether bond, an ester group, an acetyl group, a benzoyl group, an alkyl alcohol group, an amide group, or a nitrile group.

In formula (2), R³ represents an aldehyde group (-CHO) or an ester group.

### Difluoro Compound Represented by Formula (1) (Substrate, Raw Material Compound)

The substrate for use is a difluoro compound having the structure represented by formula (1).

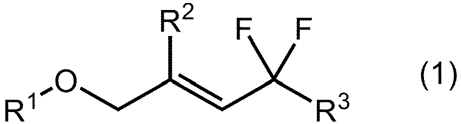

In formula (1), R¹ and R² may be the same or different, and each represents a hydrogen atom, an alkyl group, an aryl group, a group having an ether bond, an ester group, an acetyl group (CH₃CO-), a benzoyl group (C₆H₅-C(=O)-), an alkyl alcohol group, an amide group, or a nitrile group (-C≡N).

In formula (1), R³ represents an aldehyde group or an ester group.

### Organic Solvent

The organic solvent is preferably at least one organic solvent selected from the group consisting of aprotic polar solvents, halogen solvents, ether solvents, ester solvents, hydrocarbon solvents, and aromatic solvents.

The aprotic polar solvents are preferably acetone, acetonitrile, N,N-dimethylformamide (DMF), dimethylsulfoxide (DMSO), and the like.

The halogen solvents are preferably dichloromethane (CH₂Cl₂), trichloromethane (CHCl₃), and the like.

The ether solvents are preferably tetrahydrofuran (THF), dioxane, diethyl ether (Et₂O), isopropyl ether (IPE), and the like.

The ester solvents are preferably ethyl acetate (AcOEt) and the like.

The hydrocarbon solvents are preferably n-hexane and the like.

The aromatic solvents are preferably toluene and the like.

The organic solvents are more preferably aprotic polar solvents, ethyl acetate, toluene, and the like.

The amount of the organic solvent used is adjusted so that the substrate concentration (M (mol/L)) is preferably 0.01M to 5M, and more preferably 0.1M to 2M.

### Oxidizing Agent

The oxidizing agent for use is preferably a peroxide, and more preferably m-chloroperoxybenzoic acid (3-chloroperoxybenzoic acid, mCPBA).

The amount of the oxidizing agent used is preferably 0.1 to 20 equivalents, and more preferably 0.9 to 5 equivalents, in molar ratio relative to the substrate (when the substrate is taken as 1 equivalent).

### Step of Producing Difluoro Compound Represented by Formula (2)

A difluoro compound having the structure represented by formula (2), which is the target compound, can be obtained from the difluoro compound represented by formula (1), which is a substrate (raw material compound).

The reaction liquid is preferably basic with a pH of about 7 to 13. The pH of the reaction liquid is preferably adjusted using a base, such as sodium hydrogen carbonate (sodium bicarbonate, NaHCO₃) or disodium hydrogen phosphate (Na₂HPO₄). The reaction temperature is preferably 0°C to 90°C, and more preferably 0°C to 60°C. The reaction temperature is more preferably raised from a low temperature to 40°C.

The reaction time is preferably 0.5 hours to 120 hours, and more preferably 10 hours to 60 hours.

### [2-3] Method for Producing Difluoro Compound Represented by

### Formula (3)

According to the following production method, a difluoro compound having the structure represented by formula (3) can be obtained from a difluoro compound represented by formula (2). In this production method, thermodynamic control and kinetic control are appropriately used, thereby enabling the control of the production of 4-membered ring compounds and 5-membered ring compounds, satisfactorily providing a difluoro compound having the structure represented by formula (3).

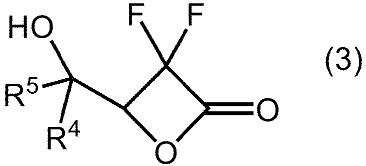

In formula (3), R⁴ and R⁵ may be the same or different, and each represents a hydrogen atom, an alkyl group, an aryl group, a group having an ether bond, an ester group, an acetyl group, a benzoyl group, an alkyl alcohol group, an amide group, or a nitrile group.

### Difluoro Compound Represented by Formula (2) (Substrate, Raw Material Compound)

The substrate for use is a difluoro compound having the structure represented by formula (2).

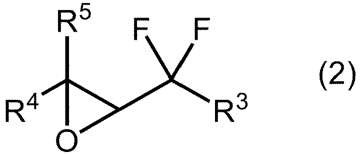

In formula (2), R⁴ and R⁵ may be the same or different, and each represents a hydrogen atom, an alkyl group, an aryl group, a group having an ether bond, an ester group, an acetyl group, a benzoyl group, an alkyl alcohol group, an amide group, or a nitrile group.

In formula (2), R³ represents an aldehyde group (-CHO) or an ester group.

### Solvent

The solvent for use is preferably water.

The solvent for use is preferably an organic solvent.

The organic solvent is preferably at least one organic solvent selected from the group consisting of aprotic polar solvents, halogen solvents, ether solvents, ester solvents, hydrocarbon solvents, and aromatic solvents.

The aprotic polar solvents are preferably acetone, acetonitrile, N,N-dimethylformamide (DMF), dimethylsulfoxide (DMSO), and the like.

The halogen solvents are preferably dichloromethane (CH₂Cl₂), trichloromethane (CHCl₃), and the like.

The ether solvents are preferably tetrahydrofuran (THF), dioxane, diethyl ether (Et₂O), isopropyl ether (IPE), and the like.

The ester solvents are preferably ethyl acetate (AcOEt) and the like.

The hydrocarbon solvents are preferably n-hexane and the like.

The aromatic solvents are preferably toluene and the like.

The organic solvent is more preferably THF or the like.

The solvent for use is preferably a mixed solvent of alkali (NaOH) + water/THF.

When a mixed solvent of water/THF is used as a solvent, the mixing ratio (the percentage of water in a mixed solvent of H₂O/THF = water/THF) is preferably 0.1% by mass to 99.9% by mass, more preferably 1% by mass to 50% by mass, and even more preferably 5% by mass to 20% by mass.

The amount of the solvent used is adjusted so that the substrate concentration (reaction concentration) (M (mol/L)) is preferably 0.01 mol/L to 10 mol/L, more preferably 0.01 mol/L to 5 mol/L, and even more preferably 0.01 mol/L to 2 mol/L.

### Alkali

The alkali for use is one having strong basicity. Preferably, the alkali for use is an agent such as sodium hydroxide (NaOH), potassium hydroxide (KOH), cesium hydroxide (CsOH), or barium hydroxide (Ba(OH)2).

The amount of alkali used (M (mol/L)) in a solvent is preferably 0.1M to 10M, more preferably 0.2M to 5M, and even more preferably 0.2M to 2M.

### Step of Producing Difluoro Compound Represented by Formula (3)

From a difluoro compound represented by formula (2), which is a substrate (raw material compound), a difluoro compound having the structure represented by formula (3), which is the target compound, can be obtained. The reaction temperature is preferably -20°C to 80°C, more preferably -20°C to 50°C, and even more preferably, more preferably, -20°C to 20°C.

The reaction time is preferably 0.5 hours to 70 hours, and more preferably 10 hours to 60 hours.

### [3] Application Examples

An application example is that a difluoro compound having the structure represented by formula (4) can be produced from a difluoro compound represented by formula (2). In this production method, the concentration of alkali (NaOH) is adjusted to enable epoxy cyclization, thereby satisfactorily producing a difluoro compound having the structure represented by formula (4) .

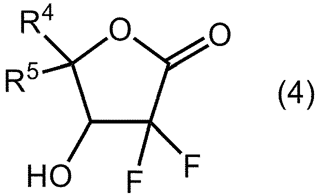

In formula (4), R⁴ and R⁵ may be the same or different, and each represents a hydrogen atom, an alkyl group, an aryl group, a group having an ether bond, an ester group, an acetyl group, a benzoyl group, an alkyl alcohol group, an amide group, or a nitrile group.

### Difluoro Compound Represented by Formula (2) (Substrate, Raw Material Compound)

The substrate for use is a difluoro compound having the structure represented by formula (2).

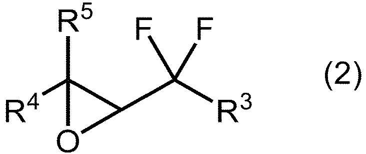

In formula (2), R⁴ and R⁵ may be the same or different, and each represents a hydrogen atom, an alkyl group, an aryl group, a group having an ether bond, an ester group, an acetyl group, a benzoyl group, an alkyl alcohol group, an amide group, or a nitrile group.

In formula (2), R³ represents an aldehyde group (-CHO) or an ester group.

### Solvent

The solvent for use is preferably water.

The solvent for use is preferably an organic solvent.

The organic solvent is preferably at least one organic solvent selected from the group consisting of aprotic polar solvents, halogen solvents, ether solvents, ester solvents, hydrocarbon solvents, and aromatic solvents.

The aprotic polar solvents are preferably acetone, acetonitrile, N,N-dimethylformamide (DMF), dimethylsulfoxide (DMSO), and the like.

The halogen solvents are preferably dichloromethane (CH₂Cl₂), trichloromethane (CHCl₃), and the like.

The ether solvents are preferably tetrahydrofuran (THF), dioxane, diethyl ether (Et₂O), isopropyl ether (IPE), and the like.

The ester solvents are preferably ethyl acetate (AcOEt) and the like.

The hydrocarbon solvents are preferably n-hexane and the like.

The aromatic solvents are preferably toluene and the like.

The organic solvent is more preferably THF or the like.

The solvent for use is preferably a mixed solvent of alkali (NaOH) + water/THF.

When a mixed solvent of water/THF is used as a solvent, the mixing ratio (the percentage of water in a mixed solvent of H₂O/THF = water/THF) is preferably 0.1% by mass to 99.9% by mass, more preferably 1% by mass to 50% by mass, and even more preferably 5% by mass to 20% by mass.

The amount of the solvent used is adjusted so that the substrate concentration (reaction concentration) (M (mol/L)) is preferably 0.1 mol/L to 10 mol/L, more preferably 0.5 mol/L to 5 mol/L, and even more preferably 0.5 mol/L to 3 mol/L. The amount of the solvent for use is preferably adjusted so as to dissolve the substrate at its saturation concentration.

### Alkali

The alkali for use is one having strong basicity. Preferably, the alkali for use is an agent such as sodium hydroxide (NaOH), potassium hydroxide (KOH), cesium hydroxide (CsOH), or barium hydroxide (Ba(OH)₂).

The amount of alkali used (M (mol/L)) in a solvent is preferably 0.1M to 10M, more preferably 0.2M to 5M, and even more preferably 0.2M to 2M.

### Step of Producing Difluoro Compound Represented by Formula (4)

From a difluoro compound represented by formula (2), which is a substrate (raw material compound), a difluoro compound having the structure represented by formula (4), which is the target compound, can be obtained. The reaction temperature is preferably 20°C to 100°C, more preferably 20°C to 80°C, and even more preferably, more preferably 30°C to 50°C.

The reaction time is preferably 0.5 hours to 70 hours, and more preferably 10 hours to 60 hours.

An application example of the production method of the present invention is that diol compounds, aldol compounds, allyl compounds, etc. can be further produced with high enantioselectivity from the fluorinated amino acid compound prepared according to the production method of the present invention.

According to the production method of the present invention, epoxidation of a disubstituted alkene also proceeds smoothly. As a result, products even as final compounds, such as a four-membered ring compound or a five-membered ring compound, can be obtained.

In the production method of the present invention, an aldehyde having difluoro at the α-position and a double bond or epoxy at the β-position can be easily derived into a four-membered ring compound or a five-membered ring compound. These compounds can serve as precursors for raw materials of medium molecules.

In the production method of the present invention, particularly when the γ-position is a disubstituted alkene, derivatization into a four-membered ring compound or a five-membered ring compound gives a quaternary carbon. These compounds can be very useful precursors of nucleotides, nucleosides, nucleic acid amidites, or nucleic acids.

Embodiments of the present invention are described above; however, the present invention is not limited to these embodiments. Needless to say, the present invention can be performed in various forms without departing from the spirit and concept of the invention.

### Examples

Embodiments of the present invention are described in more detail below based on Examples.

The present invention is not limited to these Examples.

### [1] Method for Producing Difluoro Compound Represented by Formula (1)

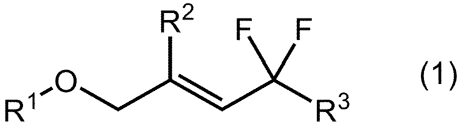

In formula (1), R¹ and R² may be the same or different, and each represents a hydrogen atom, an alkyl group, an aryl group, a group having an ether bond, an ester group, an acetyl group (CH₃CO-), a benzoyl group (C₆H₅-C(=O)-), an alkyl alcohol group, an amide group, or a nitrile group (-C≡N).

In formula (1), R³ represents an aldehyde group or an ester group.

A difluoro compound having the structure represented by formula (1) was obtained from a β-unsaturated aldehyde compound or a β-unsaturated ester compound as a substrate by combining the known Parikh-Doering oxidation and Pinnick oxidation.

### [2] Method for Producing Difluoro Compound Represented by Formula (2)

A difluoro compound having the structure represented by the following formula (2) was produced using a difluoro compound represented by formula (1) obtained according to the production method of section [1] above as a substrate.

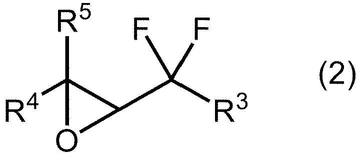

In formula (2), R⁴ and R⁵ may be the same or different, and each represents a hydrogen atom, an alkyl group, an aryl group, a group having an ether bond, an ester group, an acetyl group, a benzoyl group, an alkyl alcohol group, an amide group, or a nitrile group.

In formula (2), R³ represents an aldehyde group (-CHO) or an ester group.

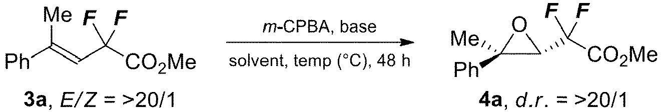

The difluoro compound produced is represented by formula (2), in which R⁴ is a phenyl group (Ph, C₆H₅-), R⁵ is a methyl group (Me, CH₃-), and R³ is a methyl ester group (-COOMe, -COOCH₃).

**Table 1**

| Table 1 | | | | | |
|---|---|---|---|---|---|
| Example | Solvent | Equivalent of Oxidizing Agent (m-CPBA) Relative to Substrate | Base | Reaction Temperature (°C) | Yield (%) |
| 1-1 | MePh | 1.5 | - | r.t. | 71 |
| 1-2 | MePh | 2.0 | Na₂CO₃ | 40 | 56 |
| 1-3 | MePh | 2.0 | NaHCO₃ | 40 | 77 |
| 1-4 | MePh | 2.0 | Na₂HPO₄ | 40 | 75 |
| 1-5 | MePh | 2.0 | K₂HPO₄ | 40 | 59 |
| 1-6 | MePh | 2.0+1.0 | NaHCO₃ | 40 | 82 |
| | MePh: Toluene | | | r.t.: Room Temperature | |

Oxidizing agent: mCPBA (m-chloroperoxybenzoic acid) was used.
Base: bases with a pH of 7 and 13 were used. The reaction proceeded smoothly by using a base, in particular, NaHCO₃ or Na₂HPO₄, and produced the target compound with high yields.
Solvent: an aprotic solvent, such as toluene, was used. The reaction proceeded smoothly by using a solvent, such as benzene, chlorobenzene, toluene, or acetic acid ethyl ester, and produced the target compound with high yields.
Reaction temperature: the reaction proceeded smoothly by raising the temperature from a low temperature to 40°C and produced the target compound from the substrate with high yields.

### [3] Method for Producing Difluoro Compound Represented by Formula (3)

A difluoro compound having the structure represented by the following formula (3) was produced using a difluoro compound represented by formula (2) obtained according to the production method of section [2] above as a substrate.

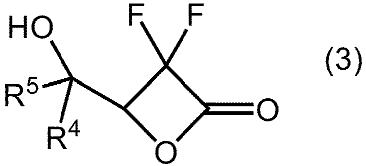

In formula (3), R⁴ and R⁵ may be the same or different, and each represents a hydrogen atom, an alkyl group, an aryl group, a group having an ether bond, an ester group, an acetyl group, a benzoyl group, an alkyl alcohol group, an amide group, or a nitrile group.

Solvent: a mixed solvent of NaOH + water/THF was used.
H₂O/THF ratio: the percentage of water in the mixed solvent of water/THF was adjusted to 20% by mass.
NaOH (alkali) concentration: the concentration was adjusted to 0.2M.
Reaction concentration: The amount of the solvent used was adjusted to give 2 mol/L as a concentration of the substrate.
Reaction temperature: the temperature was adjusted to -20°C.

**Table 2**

| Table 2 | | | | |
|---|---|---|---|---|
| Example | R⁴ | R⁵ | Reaction Time (h) | Yield (%) |
| 2-1 | C₆H₅- | Me | 48 | 20 |
| 2-2 | 4-F-C₆H₄- | Me | 48 | 50 |
| 2-3 | 4-Me-C₆H₄- | Me | 9 | 49 |
| 2-4 | 2-MeO-C₆H₄- | Me | 48 | 46 |
| 2-5 | 2-naphthyl- | Me | 31 | 42 |
| 2-6 | BnOCH₂- | Me | 42 | 21 |

| | | | | |
|---|---|---|---|---|
| Me: Methyl Group MeO: Methoxy Group Bn: Benzyl Group | | | | |

The reaction proceeded smoothly and produced the target compound from the substrate with high yields.

### [4] Application Example

A difluoro compound having the structure represented by the following formula (4) was produced using a difluoro compound represented by formula (2) obtained according to the production method of section [2] above as a substrate.

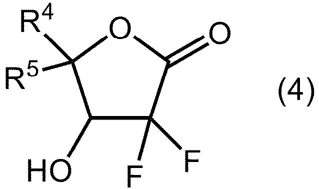

In formula (4), R⁴ and R⁵ may be the same or different, and each represents a hydrogen atom, an alkyl group, an aryl group, a group having an ether bond, an ester group, an acetyl group, a benzoyl group, an alkyl alcohol group, an amide group, or a nitrile group.

Solvent: a mixed solvent of NaOH + water/THF was used.
H₂O/THF ratio: the percentage of water in the mixed solvent of water/THF was adjusted to 20% by mass.
NaOH (alkali) concentration: the concentration was adjusted to 0.2M.
Reaction concentration: the amount of the solvent used was adjusted to give 3 mol/L (saturation concentration of the substrate) as a concentration of the substrate.
Reaction temperature: the temperature was adjusted to 30°C.

**Table 3**

| Table 3 | | | | |
|---|---|---|---|---|
| Example | R⁴ | R⁵ | Reaction Time (h) | Yield (%) |
| 3-1 | C₆H₅- | Me | 48 | 72 |
| 3-2 | 4-F-C₆H₄- | Me | 48 | 47 |
| 3-3 | 4-Cl-C₆H₄- | Me | 48 | 45 |
| 3-4 | 4-Br-C₆H₄- | Me | 48 | 50 |
| 3-5 | 4-Me-C₆H₄- | Me | 9 | 30 |
| 3-6 | 3-MeO-C₆H₄- | Me | 48 | 43 |
| 3-7 | 2-MeO-C₆H₄- | Me | 48 | 27 |
| 3-8 | 2-naphthyl- | Me | 31 | 20 |
| 3-9 | C₆H₅- | Et | 31 | 53 |
| 3-10 | BnOCH₂- | Me | 42 | 62 |

| | | | | |
|---|---|---|---|---|
| Me: Methyl Group Et: Ethyl Group MeO: Methoxy Group Bn: Benzyl Group | | | | |

The reaction proceeded smoothly and produced the target compound from the substrate with high yields.

### Industrial Applicability

According to the production method of the present invention, epoxidation of a disubstituted alkene also proceeds smoothly. As a result, products even as final compounds, such as a four-membered ring compound or a five-membered ring compound, can be obtained.

In the production method of the present invention, an aldehyde having difluoro at the α-position and a double bond or epoxy at the β-position can be easily derived into a four-membered ring compound or a five-membered ring compound. These compounds can serve as precursors for raw materials of medium molecules.

In the production method of the present invention, particularly when the γ-position is a disubstituted alkene, derivatization into a four-membered ring compound or a five-membered ring compound gives a quaternary carbon. These compounds can be very useful precursors of nucleotides, nucleosides, nucleic acid amidites, or nucleic acids.

## Claims

1. A compound represented by the following formula (1): 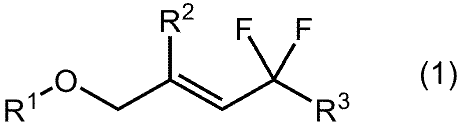 wherein
R¹ and R² may be the same or different, and each represents an alkyl group, an aryl group, a group having an ether bond, an ester group, an acetyl group, a benzoyl group, an alkyl alcohol group, an amide group, or a nitrile group, and
R³ represents an aldehyde group or an ester group.

2. A compound represented by the following formula (2): 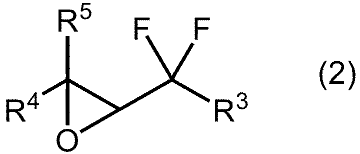 wherein
R⁴ and R⁵ may be the same or different, and each represents a hydrogen atom, an alkyl group, an aryl group, a group having an ether bond, an ester group, an acetyl group, a benzoyl group, an alkyl alcohol group, an amide group, or a nitrile group, and
R³ represents an aldehyde group or an ester group.

3. A compound represented by the following formula (3): 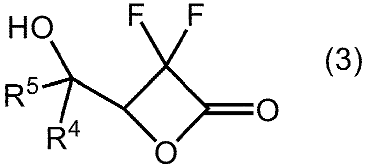 wherein
R⁴ and R⁵ may be the same or different, and each represents a hydrogen atom, an alkyl group, an aryl group, a group having an ether bond, an ester group, an acetyl group, a benzoyl group, an alkyl alcohol group, an amide group, or a nitrile group.
